(19) Europäisches Patentamt

European Patent Office

Office européen des brevets

(11) **EP 1 561 640 A2**

(12) **EUROPÄISCHE PATENTANMELDUNG**

(43) Veröffentlichungstag:
10.08.2005 Patentblatt 2005/32

(51) Int Cl.⁷: **B60Q 3/02**, F21V 9/10

(21) Anmeldenummer: 05002444.7

(22) Anmeldetag: 04.02.2005

(84) Benannte Vertragsstaaten:
AT BE BG CH CY CZ DE DK EE ES FI FR GB GR HU IE IS IT LI LT LU MC NL PL PT RO SE SI SK TR
Benannte Erstreckungsstaaten:
AL BA HR LV MK YU

(30) Priorität: 06.02.2004 US 541915 P

(71) Anmelder: **Goodrich Hella Aerospace Lighting Systems GmbH**
**59557 Lippstadt (DE)**

(72) Erfinder:
• **Beermann, Franz-Josef**
**59581 Warstein (DE)**
• **Scheithauer, Ulrich**
**59597 Erwitte (DE)**

• **Broelemann, Rolf**
**33154 Salzkotten (DE)**
• **Geske, Carsten**
**59302 Oelde (DE)**
• **Schulz, Rico**
**59558 Rixbeck (DE)**
• **Mueller, Bernhard**
**59823 Arnsberg (DE)**
• **Gronemeier, Frank**
**59199 Boenen (DE)**
• **Roebke, Steffen**
**33098 Paderborn (DE)**

(74) Vertreter: **Hilleringmann, Jochen, Dipl.-Ing. et al**
**Patentanwälte**
**von Kreisler-Selting-Werner,**
**Bahnhofsvorplatz 1 (Deichmannhaus)**
**50667 Köln (DE)**

(54) **Farblicht für die Passagiere eines Personentransportmittels, insbesondere für die Kabine eines Flugzeuges**

(57) Das Farblicht für die Passagiere eines Personentransportmittels, insbesondere für die Kabine eines
Flugzeuges, ist versehen mit mehreren Leuchtmitteln,
von denen jedes elektromagnetische Wellen in einem
schmalbandigen Wellenlängenbereich abstrahlt, wobei
sich die Wellenlängenbereiche vorzugsweise nicht
überlappen. Ferner ist das Farblicht versehen mit einer
Ansteuereinheit zum Ansteuern der Leuchtmittel derart,
dass über das Farbempfinden des menschlichen Auges
und der dadurch ausgelösten (bio-)chemischen Prozesse im Körper eine auf die Passagiere beruhigende oder
anregende Wirkung erzielbar ist.

EP 1 561 640 A2

**Beschreibung**

Die Erfindung betrifft ein Farblicht für die Passagiere eines Personentransportmittels, bei dem es sich insbesondere um ein Flugzeug handelt. Das Farblicht wird für die Beleuchtung im Passagierraum des Transportmittels, im Falle eines Flugzeuges also für die Beleuchtung der Kabine eingesetzt.

Zur Schaffung einer angenehmen Umgebung in Räumlichkeiten ist es bekannt, farbiges Licht, aber auch farblich wechselndes Licht einzusetzen (sogenanntes Moodlight). Es ist bekannt, derartige Farblichteffekte durch entsprechende Ansteuerung von GRB-Leuchtmitteln zu erzeugen. Als Leuchtmittel eignen sich hier insbesondere LEDs.

Eine Aufgabe der Erfindung ist es, durch Verwendung farbiger Leuchtmittel bestimmter Wellenlängen und/oder Anordnungen eine optimale Farbwiedergabe zu erzielen und das menschliche Farbempfinden zu steigern.

Zur Lösung dieser Aufgabe wird mit der Erfindung ein Farblicht für die Passagiere eines Personentransportmittels, insbesondere für die Kabine eines Flugzeuges, vorgeschlagen, wobei das Farblicht versehen ist mit

- mehreren Leuchtmitteln, von denen jedes elektromagnetische Wellen in einem schmalbandigen Wellenlängenbereich abstrahlt, wobei sich die Wellenlängenbereiche vorzugsweise nicht überlappen und
- einer Ansteuereinheit zum Ansteuern der Leuchtmittel derart, dass über das Farbempfinden des menschlichen Auges und der dadurch ausgelösten (bio-)chemischen Prozesse im Körper eine auf die Passagiere beruhigende oder anregende Wirkung erzielbar ist.

Durch intensive Untersuchungen hat man herausgefunden, dass der menschliche Körper insbesondere durch spezielle Frequenzen/Wellenlängen des auf den Körper auftreffenden Lichts besonders effektiv beeinflussbar ist, was seine Gemütsstimmung und sein Wohlbefinden anbelangt. Mit dem erfindungsgemäßen Farblicht wird gerade dieser optimierte Prozess der bewussten Stimulation bzw. bewussten Nicht-Stimulation der Passagiere ausgenutzt.

Bei der Kombination besonders schmalbandiger Leuchtmittel ist es nicht möglich, durch Überlagerung der Wellenlängen Farbtemperaturen zu erzielen, die eine gute Farbwiedergabe angestrahlter Gegenstände ergeben. Unter Berücksichtigung der Wirkweise der Rezeptoren des menschlichen Auges und angepasst an den Farbkreis gelingt es, mit der Auswahl einiger weniger schmalbandiger Leuchtmittel, vorzugsweise Leuchtdioden, eine sehr gute Farbwiedergabe zu erzielen. Diese Farbwiedergabe ist auf das spezielle Farbempfinden des menschlichen Auges und der dadurch ausgelösten (bio-)chemischen Prozesse im Körper abgestimmt, und zwar dergestalt, dass beispielsweise eine beruhigende oder eine anregende Wirkung auf die Personen ausgeübt wird.

Durch die Verwendung der erfindungsgemäß schmalbandig abstrahlenden Leuchtmittel kann das menschliche Farbempfinden unterstützt werden. Neben rotes, grünes und blaues Licht abstrahlenden schmalbandigen Leuchtmitteln können auch noch zusätzlich in einem kleinen Raumwinkelbereich, wie er beispielsweise bei LEDs üblich ist, oder auch in einem größeren Raumwinkelbereich abstrahlende weiße Lichtquellen verwendet werden. Unter einem spektral schmalbandig abstrahlenden Leuchtmittel ist im Rahmen dieser Erfindung eine Lichtquelle zu verstehen, die innerhalb eines Wellenlängenbereichs von bis zu 20 nm, vorzugsweise bis zu 10 nm und insbesondere bis zu 5 nm abstrahlt. Ein räumlich schmalbandig abstrahlendes Leuchtmittel ist beispielsweise eine Lichtquelle, die in einem Raumwinkelbereich von bis zu beispielsweise 120 ° abstrahlt.

Zweckmäßigerweise umfassen die Leuchtmittel gruppenweise angeordnete Lichtquellen, wobei die Lichtquellen jeder Gruppe in unterschiedlichen Wellenlängen bzw. spektral schmalbandigen Wellenlängenbereichen liegt abstrahlen. Diesen Leuchtmitteln können zweckmäßigerweise ein oder mehrere weißlichtaussendende Lichtquellen hinzugefügt werden. Auf diese Weise werden also bestimmte Wellenlängen bzw. Wellenlängenbereiche des weißen Lichts verstärkt bzw. betont.

Bei der Kombination spektral schmalbandig abstrahlender Leuchtmittel, wie es bei LEDs der Fall ist, ist es nicht möglich, durch Überlagerung der Wellenlängen eine Emission zu erzielen, welche eine gute Farbwiedergabe ermöglicht. Unter Berücksichtigung der Physiologie des menschlichen Auges und angepasst an die Definition der Farbwiedergabe selbst gelingt es erfindungsgemäß überraschenderweise mit der Auswahl einiger spezieller ebenfalls spektral schmalbandiger Leuchtmittel, vorzugsweise LEDs, eine gute Farbwiedergabe zu erreichen. Gemäß einer ersten Version des erfindungsgemäßen Farblichts weist dieses blaues Licht von 470 nm ± 0 - 10 nm, grünes Licht von 540 nm ± 0 - 10 nm, gelbes Licht von 580 nm ± 0 - 10 nm und rotes Licht von 615 ± 0 - 10 nm auf. Die zuvor angegebenen Wellenlängenbereiche erstrecken sich vorzugsweise um ± 0 - 5 nm und insbesondere ± 0 - 2,5 nm um die jeweiligen Mittenwellenlängen. Eine erfindungsgemäße optimierte Version des Farblichts weist blaues Licht von 450 nm ± 0 - 5 nm und 470 nm ± 0 - 5 nm, grünes Licht von 450 nm ± 0 - 10 nm, gelbes Licht von 580 nm ± 0 - 10 nm und rotes Licht von 610 nm ± 0 - 5 nm und 625 nm ± 0 - 5 nm auf.

Neben der verbesserten Farbwiedergabe nach der Erfindung kann durch hinzufügen anderer spezieller Wellenlängen auf die Physiologie des Menschen Einfluss genommen werden. So kann beispielsweise durch Hinzufügen von spektral schmalbandigem blauen Licht in einem Wellenlängenbereich zwischen 450 nm und 465 nm die Melatonin-Ausschüttung und damit der Schlaf unterdrückt werden. Die damit verbundene Steuerung des Wach-Schlaf-Rhythmus ermöglicht eine aktive Verringerung der Auswirkungen des Syndroms der Zeitverschiebung (sogenanntes Jetlag).

Mit Leuchtmittel im Sinne dieser Erfindung sind auch Einrichtungen gemeint, die evtl. zumindest lediglich teilweise elektromagnetische Wellen im nichtsichtbaren Wellenlängenbereich aussenden.

Die schmalbandigen Leuchtmittel verfügen zweckmäßigerweise über folgende Spitzen-Wellenlängen. In einer Basisversion für ausreichende Farbwiedergabe und dynamische Farbverläufe liegen die Spitzen-Wellenlängen bei 450 nm und 550 nm sowie 580 nm und 610 nm. Die Leuchtmittel sind gruppenweise entsprechend der nachfolgend aufgeführten Wellenlängenangaben nebeneinander angeordnet:

$$(450 \text{ nm} + 550 \text{ nm}) + (450 \text{ nm} + 550 \text{ nm}) + 580 \text{ nm} + 610 \text{ nm}.$$

Eine Version mit optimierter Farbwiedergabe und dynamischen Farbverläufen weist, in der nachfolgend angegebenen Reihenfolge gruppenweise angeordnete Leuchtmittel mit den folgenden Spitzen-Wellenlängen auf:

$$(450 \text{ nm} + 550 \text{ nm}) + 580 \text{ nm} + (450 \text{ nm} + 550 \text{ nm}) + 610 \text{ nm}$$

$$+ (450 \text{ nm} + 550 \text{ nm}) + 590 \text{ nm} + (450 \text{ nm} + 550 \text{ nm}) + 620 \text{ nm}.$$

Eine Moodlight-Version mit optimierter Farbwiedergabe und zusätzlichem Aufwachlicht in dynamischen Farbverläufen lässt sich unter Hinzufügung weiterer Wellenlängen, die kleiner als 450 nm sind, realisieren. Durch Hinzufügen derartiger Wellenlängen zum ausgestrahlten Licht können mit entsprechenden Steuerungsprogrammen Aufwach- oder Aktivierungsphasen lichttechnisch angeregt und unterstützt werden. Beispielsweise behindert blaues Licht mit einer Wellenlänge von 450 nm die Melatonin-Ausschüttung im menschlichen Körper und unterdrückt somit (im begrenzten Umfang) Schlaf. Die Steuerung des Lichts, das heißt deren Programmstart und -ablauf kann durch die Kabinencrew direkt oder automatisch infolge des jeweiligen "Standorts" des Flugzeuges im Flug durch entsprechende Positionssignale zum Beispiel entsprechend dem GPS, erfolgen. Auf diese Weise können Tag- und Nachtphasen durch entsprechende Lichteinkopplung in die Kabine simuliert werden. Selbiges gilt auch für die zeitliche Steuerung von Einschlaf- und Aufwachphasen.

## Patentansprüche

1.    Farblicht für die Passagiere eines Personentransportmittels, insbesondere für die Kabine eines Flugzeuges, mit

   -    mehreren Leuchtmitteln, von denen jedes elektromagnetische Wellen in einem schmalbandigen Wellenlängenbereich abstrahlt, wobei sich die Wellenlängenbereiche vorzugsweise nicht überlappen, und
   -    einer Ansteuereinheit zum Ansteuern der Leuchtmittel derart, dass über das Farbempfinden des menschlichen Auges und der **dadurch** ausgelösten (bio-)chemischen Prozesse im Körper eine auf die Passagiere beruhigende oder anregende Wirkung erzielbar ist.

2.    Farblicht nach Anspruch 1, **dadurch gekennzeichnet, dass** die Leuchtmittel in mehreren Gruppen angeordnet sind, wobei die Leuchtmittel pro Gruppe in unterschiedlichen schmalbandigen Wellenlängenbereichen abstrahlen.

3.    Farblicht nach Anspruch 1 oder 2, **dadurch gekennzeichnet, dass** die Leuchtmittel auch solche umfassen, die breitbandig und insbesondere weißes Licht ausstrahlen.

4.    Farblicht nach Anspruch 1 bis 3, **dadurch gekennzeichnet, dass** die Leuchtmittel blaues Licht von 470 nm $\pm$ 0 - 10 nm, grünes Licht von 540 nm $\pm$ 0 - 10 nm, gelbes Licht von 580 nm $\pm$ 0 - 10 nm und rotes Licht von 615 nm $\pm$ 0 - 10 nm abstrahlen.

5.    Farblicht nach Anspruch 1 bis 3, **dadurch gekennzeichnet, dass** die Leuchtmittel blaues Licht von 450 nm $\pm$ 0 - 5 nm und 470 nm $\pm$ 0 - 5 nm, grünes Licht von 540 nm $\pm$ 0 - 10 nm, gelbes Licht von 580 nm $\pm$ 0 - 10 nm und rotes Licht von 610 nm $\pm$ 0 - 5 nm und 625 nm $\pm$ 0 - 5 nm abstrahlen.

6.    Farblicht nach Anspruch 1 bis 5, **dadurch gekennzeichnet, dass** die Leuchtmittel LED' s aufweisen

7.    Farblicht nach Anspruch 1 bis 6, **dadurch gekennzeichnet, dass** die Ansteuerung der Leuchtmitel in Abhängigkeiten der Tageszeit am jeweiligen Ort des Personentransportmittels erfolgt.